(19) Europäisches Patentamt

European Patent Office

Office européen des brevets

(11) EP 0 956 871 A2

(12) **EUROPÄISCHE PATENTANMELDUNG**

(43) Veröffentlichungstag:
17.11.1999 Patentblatt 1999/46

(51) Int. Cl.⁶: **A61M 1/16**

(21) Anmeldenummer: 99107242.2

(22) Anmeldetag: 14.04.1999

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 06.05.1998 DE 19820158

(71) Anmelder: Convergenza AG
9490 Vaduz (LI)

(72) Erfinder:
• Plechinger, Hans
Cranbrook, BC VIC 6H3 (CA)

• Stevens, Klaus
52152 Simmerath (DE)
• Brockhoff, Alexander
9494 Schaan (LI)

(74) Vertreter:
Vetter, Ewald Otto, Dipl.-Ing. et al
Meissner, Bolte & Partner
Anwaltssozietät
Postfach 10 26 05
86016 Augsburg (DE)

(54) **Blutoxygeniervorrichtung**

(57) Vorrichtung zur Blutoxygenierung durch Sauerstoffübertragung von einem Gasstrom (60) durch eine gasdurchlässige, jedoch flüssigkeitsundurchlässige Gasauschtautausch-Membran (16) in einen Blutstrom (62). Der Druck des Sauerstoff-Gasstromes wird entlang der Gasaustausch-Membran (16) in Abhängigkeit vom Blutdruck auf einem an den Druck des Blutstromes angenäherten Wert gehalten. Eine Gasdruckeinstellvorrichtung (124) hat einen Steuermembrankörper (136), der Teil einer Wand eines Gehäuses (103) ist, in welchem die Gesamtaustausch-Membran (16) angeordnet ist.

FIG.1

EP 0 956 871 A2

**Beschreibung**

Die Erfindung betrifft eine Blutoxygeniervorrichtung gemäß den Oberbegriffen von Anspruch 1 und 4.

Eine Blutoxygeniervorrichtung dieser Art ist aus der EP 0 373 847 A2 bekannt. Weitere Vorrichtungen zur Blutoxygenierung sind aus den DE 43 20 198 C1, US 3 525 481 und WO 94/03265 (PCT/US93/07165) bekannt.

In Oxygenatoren sind ein Gasstrom, der aus Sauerstoff angereichertem Gas oder vorzugsweise aus reinem Sauerstoff besteht, und ein Blutstrom durch eine Gas-durchlässige, jedoch Blut-undurchlässige Gasaustausch-Wand, meistens Gasaustausch-Membran genannt, voneinander getrennt. Durch die Gasaustausch-Wand hindurch kann Sauerstoff vom Gastrom in den Blutstrom, und umgekehrt Kohlendioxyd vom Blutstrom in den Gasstrom diffundieren, ohne daß Blut vom Blutstrom in den Gasstrom gelangt. Dabei dürfen in dem Blutstrom keine Sauerstoffbläschen oder Luftbläschen entstehen, weil dies zu Embolien und zum Absterben von Gehirnzellen in einem Patienten führen kann, welchem das oxygenierte Blut zugeführt wird. In den bekannten Oxygenatoren hat der Sauerstoff-Gasstrom einen geringen Druck, welcher nur wenig über dem Atmosphärendruck liegt. Der Druck des Blutstromes im Oxygenator hat normalerweise einen ungefähr 250 mm Hg höheren Druck. Zur Erzeugung einer möglichst hohen Sauerstoff-Transferrate ist es bekannt, Gasaustausch-Membranen in Form einer großen Vielzahl von Kapillarrohren zu verwenden, welche auf ihrer Innenseite von dem Sauerstoff-Gas durchströmt werden und welche auf ihrer Außenseite vom Blutstrom umströmt werden. Die Kapillarrohre haben für das Gas, welches durch sie hindurchströmt, nur einen sehr kleinen Strömungswiderstand. Zur Regulierung der Sauerstoff-Transferrate vom Sauerstoff-Gasstrom in den Blutstrom ist es bekannt, die Zusammensetzung des Gases zu verändern, d.h. mit Sauerstoff angereicherte Luft, wobei der Sauerstoffgehalt bis zu 100 Prozent betragen kann, und/oder die Gschwindigkeit des Gasstromes durch die Kapillarrohre zu verändern. Der Diffusionswiderstand der Kapillarrohre ist für Sauerstoff wesentlich höher (kleine Sauerstoff-Strömungsgeschwindigkeit) als für Kohlendioxyd (wesentlich höhere Kohlendioxyd-Strömungsgeschwindigkeit). Je größer die Gasaustausch-Membran (Anzahl der Kapillarrohre) ist, desto größer ist die Sauerstoff-Transferrate vom Sauerstoff-Gasstrom in den Blutstrom. Dies führt jedoch zu einem sehr wesentlichen Nachteil, welcher darin besteht, daß Blut bei Berührung mit Fremdkörpern in nachteiliger Weise verändert wird, um so mehr, je größer die vom Blut berührte Fremdkörper-Kontaktfläche ist und je länger eine solche Berührung stattfindet. Es entsteht eine Traumatisierung von Blutelementen und gleichzeitig eine Denaturierung von Blutalbuminen.

Oxygenatoren werden normalerweise als wesentlicher Teil von Herz-Lungen-Maschinen, Nieren-Dialyse-Maschinen und anderen Anlagen verwendet, die einen extrakorporalen Kreislauf bilden, welcher einem Patienten Blut entnimmt, das Blut behandelt, insbeondere mit Sauerstoff anreichert, und dann das behandelte Blut wieder dem Patienten zuführt. Je kleiner ein Patient ist, also insbesondere bei kleinen Kindern, desto wichtiger ist es, daß das Blut im extrakorporalen Kreislauf mit möglichst wenig Fremdkörper-Kontaktflächen, und auch nur auf kurze Zeit, in Berührung kommt und im oxygenierten Blut keine Gasbläschen, auch wenn sie noch so mikroklein sind, entstehen. Der extrakorporale Kreislauf muß beim Anschluß an einen Patienten mit einer Blutersatzlösung gefüllt sein. Diese vermischt sich während der Blutbehandlung mit dem Eigenblut des Patienten. Nach der Blutbehandlung muß der Patient möglichst sein gesamtes eigenes Blut wieder zurückbekommen. Hierfür wird dem Patienten nach der Blutbehandlung weitgehend die gesamte Menge des Blut-Blutersatzlösungs-Gemisches langsam zugeführt, damit der Körper des Patienten Zeit hat, die Blutersatzlösung abzubauen, so daß er nach einer gewissen Zeit im wesentlichen nur noch eigenes Blut in seinem Körper hat.

Durch die Erfindung soll die Aufgabe gelöst werden, eine Vorrichtung zum Oxygenieren von Blut derart auszubilden, daß der Gasdruck in Abhängigkeit vom Blutdruck genauer und verzögerungsfreier einstellbar ist, die Vorrichtung zuverlässig arbeitet, einen einfachen Aufbau hat und die Gasaustausch-Membran eine lange Lebensdauer hat.

Diese Aufgabe wird gemäß der Erfindung durch die kennzeichnenden Merkmale von Anspruch 1 gelöst.

Weitere Merkmale der Erfindung sind in den Unteransprüchen enthalten.

Durch die Erfindung wird der Sauerstoff-Gasdruck, welcher im Oxygenator herrscht, von ungefähr 760 mm Hg (ungefähr Atmosphärendruck) der bekannten Oxygenatoren auf den Druckwert oder bis nahe zu dem Druckwert erhöht, den das Blut im Oxygenator hat, welcher ungefähr 250 mm Hg höher ist. Der Partialdruck des Sauerstoffes im Blut beträgt ungefähr 50 mm Hg. Damit herrscht auf beiden Seiten der Gasaustausch-Membran ungefähr gleicher Druck, so daß sie nur wenig mechanisch belastet wird. Gleichzeitig hat die Erhöhung des Sauerstoff-Gassdruckes eine Erhöhung der Sauerstoff-Transferrate von ungefähr 30 Prozent zur Folge. Bei bekannten Oxygenatoren, die einen Sauerstoff-Gasdruck von ungefähr Atmosphärendruck (760 mm Hg) verwenden, ergibt sich eine Sauerstoff-Transferrate von

$$T = k \cdot \text{Delta } P = k \cdot (760 - 50) = k \cdot 710.$$

Für einen auf nahezu Blutdruck erhöhten Sauerstoff-Gasdruck in einem Blutoxygenator gemäß der Erfindung ergibt sich eine Blut-Transferrate von

$$T = k \cdot Delta\ P = k \cdot (760 + 250 - 50) = k \cdot 960.$$

k = Konstante; P = Druck

Der Wert "960" ist ungefähr 30 Prozent größper als der Wert "710".

Die Erfindung bietet eine Vorrichtung, welche einen einfachen Aufbau und nur wenige Teile hat. Die Teile haben eine lange Lebensdauer und hohe Betriebssicherheit, insbesondere die in der Vorrichtung verwendeten Membranen, da sie nur kleinen oder keinen Differenzdrücken ausgesetzt sind.

Die Erfindung wird im folgenden mit Bezug auf die Zeichnung anhand einer bevorzugten Ausführungsform als Beispiel beschrieben. In der Zeichnung zeigt

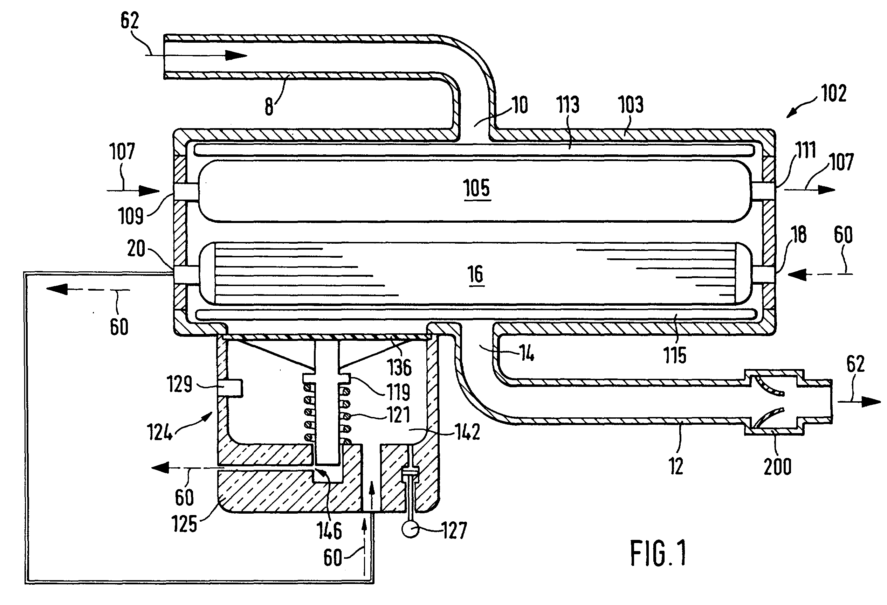

Fig. 1    schematisch einen Querschnitt einer Blutoxygeniervorrichtung nach der Erfindung.

Die in Fig.1 dargestellte Blutoxygeniervorrichtung enthält in einem Gehäuse 103 eine Gasaustausch-Membran 16 in Form eines Bündels von sehr vielen Kapillarrohren und darüber angeordnet einen Wärmetauscher 105. Ein durch gestrichelte Pfeile gekennzeichneter Gasstrom 60 strömt mit Bezug auf Fig.1 von rechts nach links auf dem Weg von einem Gaseinlaß 18 zu einem Gasauslaß 20 des Gehäuses 103 in parallelen Strömen durch die Kapillarrohre, welche die Gasaustausch-Membran 16 bilden.

Wärmetauscherflüssigkeit 107 strömt in entgegengesetzter Richtung von links nach rechts von einem Einlaß 109 zu einem Auslaß 111 durch den Wärmetauscher 105.

Der Blutstrom, gekennzeichnet durch Pfeile 62 in ausgezogenen Linien, strömt von einer Blutzufuhrleitung 8 durch einen Bluteinlaß 10 auf der Gehäuseoberseite in einen Strömungsverteiler 113 in das Gehäuse 103, dann durch Schwerkraft von oben nach unten durch den Wärmetauscher 105 und über die Oberflächen der Kapillarrohre, welche die Gasaustausch-Membran 16 bilden, und dann über einen Strömungssammler 115 durch einen Blutauslaß 14 am unteren Gehäuseende. An den Blutauslaß 14 ist die Blutabgabeleitung 12 angeschlossen.

Der Gasauslaß 20 der Kapillarrohre, welche die Gasaustausch-Membran 16 bilden, ist an eine Gaseinlaßkammer 142 eines Druckreglers 124 angeschlossen, welche durch eine flexible Membran 136 von dem Innenraum des Gehäuses 103 an der Stelle getrennt ist, wo der Blutstrom von der Gasaustausch-Membran 16 zum Blutauslaß 14 strömt. Diese Druckregler-Membran 136 bildet somit einen den Blutstrom begrenzenden Teil des Gehäuses 103. In der Gaseinlaßkammer befindet sich ein Ventilkörper oder Schieber 119, welcher von der Druckregler-Membran 136 entgegen der Kraft einer Feder 121 quer zur Öffnungsachse einer Druckregler-Gasauslaßöffnung 146 verstellbar ist, um diese quer zu ihrer Öffnungsachse mehr oder weniger weit zu verschließen oder zu öffnen. Die Druckregler-Gasauslaßöffnung 146 und der Ventilkörper 119 bilden zusammen ein Druckregelventil oder eine einstellbare Drossel, welche für den Gasstrom 60 einen größeren Strömungswiderstand bildet als die Kapillarkanäle der Kapillarrohre, welche die Gasaustausch-Membran 16 bilden. Dieser Druckregler 124 läßt den Gasdruck des Gasstromes dem Blutdruck des Blutstromes folgen, so daß der Gasdruck dem Blutdruck angenähert ist, jedoch nie über den Blutdruck steigen kann. Die Druckregler-Membran 136 ist an dem Gehäuse 103 befestigt. Ein Gehäuse 125 des Druckreglers 124 ist an dem Oxygenator-Gehäuse 103 lösbar befestigt und kann von diesem ohne die Druckregler-Membran 136 entfernt werden, zusammen mit dem Ventilkörper 119, der Druckfeder 121 und der im Druckreglergehäuse 125 gebildeten Druckregler-Gasauslaßöffnung 146. Das Druckreglergehäuse 125 kann aus durchsichtigem Material bestehen, damit optisch kontrolliert werden kann, ob der Ventilkörper 119 funktioniert oder beispielsweise klemmt. Der Druckregler kann ein Druckanzeigegerät 127 zur Anzeige des in der Gaseinlaßkammer 142 herrschenden Druckes und ein Sicherheitsventil 129 aufweisen, welches garantiert, daß der Gasdruck stets unterhalb einem vorbestimmten Grenzwert bleibt.

Stromabwärts von der Gasaustausch-Membran 16 ist nahe bei dem Blutauslaß 14 im Blutweg eine variable Blut-Strömungsdrossel 200 angeordnet. Die Blutströmungsdrossel 200 sorgt dafür, daß der Blutdruck im Oxygenator erhöht werden kann, und daß der Blutdruck mit dem Blut-Volumenstrom nicht quadratisch fällt, sondern z.B. etwa linear. Die variable Strömungsdrossel 200 hat einen sich in Abhängigkeit vom Volumenstrom ändernden Strömungswiderstand, wobei der Strömungswiderstand mit abnehmendem Volumenstrom größer wird, so daß der Druck nicht so stark sinkt wie ohne Anpassung des Strömungswiderstandes. Der Strömungsquerschnitt der variablen Strömungsdrossel 200 ist durch federn nachgiebige Elemente, vorzugsweise flexible Lippen aus Gummi, Kunststoff oder Metall gebildet, welche vom Blutdruck in Richtung auf einen größeren Strömungsquerschnitt gedrängt werden.

**Patentansprüche**

1. Blutoxygeniervorrichtung zur Sauerstoffübertragung von einem Sauerstoff-Gasstrom (60) durch eine gasdurchlässige, jedoch für flüssiges Blut undurchlässige Gasaustausch-Membran (16) in einen Blutstrom (62), enthaltend eine automatische Gasdruckeinstellvorrichtung (124) welche den Gasdruck des Gasstromes, welcher entlang der Gasaustausch-Membran (16) herrscht, in Abhängigkeit von dem Blutdruck des Blutstromes (62), welcher ebenfalls entlang der Gasaustausch-Membran (16), jedoch auf der vom Gas abgewandten Membranseite herrscht, steuert oder regelt und dabei auf einem vorbetimmten Wert hält,wobei die Gasdruckeinstellvorrichtung (124) einen gasundurchlässigen und flüssigkeitsundurchlässigen flexiblen Steuermembrankörper (136) aufweist, welcher eine dem Blutstrom ausgesetzte Seite und eine in entgegengesetzter Richtung dem Gasstrom ausgesetzte Seite aufweist,
**dadurch gekennzeichnet,**
daß der Steuermembrankörper (136) ein den Blutstrom begrenzendes Teil einer Wand eines Gehäuses (103) ist, in welchem die Gasaustausch-Membran (16) untergebracht ist, wobei die Gehäusewand und die Gasaustausch-Membran (16) zwischen sich einen Kanal für den Blutstrom bilden.

2.  Blutoxygeniervorrichtung nach Anspruch 1,
    **dadurch gekennzeichnet,**
    daß die Gasdruckeinstellvorrichtung (124) und ihr Steuermembrandkörper (136) auf der bezüglich des Blutstromes stromabwärtigen Seite der Gasaustausch-Membran (16) angeordnet sind und der Steuermembrankörper (136) dem Blut des Blutstromes (62) auf der stromabwärtigen Seite der Gasaustausch-Membran (16) ausgesetzt ist.

3.  Blutoxygeniervorrichtung nach Anspruch 1,
    **dadurch gekennzeichnet,**
    daß der Steuermembrankörper (136) dem Blut des Blutstromes (62) an einer Stelle ausgesetzt ist, wo der Blutstrom (62) von der Gasaustausch-Membran (16) zu einem Blutauslaß (14) des Gehäuses (103) strömt.

4.  Blutoxygeniervorrichtung nach einem der Ansprüche 1 bis 3,
    **dadurch gekennzeichnet,**
    daß der Steuermembrankörper (136) eine Gaseinlaßkammer (142) der Gasdruckeinstellvorrichtung (124) von dem Innenraum des Gehäuses (103) trennt, daß zwischen der Gaseinlaßkammer (142) und einer Gasauslaßöffnung (146) ein von dem Steuermembrankörper (136) betätigbares Ventil (119) angeordnet ist, und daß der Weg des Sauerstoff-Gasstromes (60) stromabwärts der Gasaustausch-Membran (16) an die Gaseinlaßkammer (142) angeschlossen ist.

5.  Blutoxygeniervorrichtung nach einem der Ansprüche 1 bis 4,
    **dadurch gekennzeichnet,**
    daß die Gasdruckeinstellvorrichtung (124) derart ausgebildet ist, daß sie den im Gehäuse (103) an der Gasaustausch-Membran (16) herrschenden Gasdruck des Gasstromes (60) in Abhängigkeit vom Druck des Blutstromes (62) an den Druck dieses Blutstromes annähert, jedoch nicht über den Druck des Blutstromes hinaus ansteigen läßt.

6.  Blutoxygeniervorrichtung nach einem der vorhergehenden Ansprüche,
    **dadurch gekennzeichnet,**
    daß die Gasdruckeinstellvorrichtung (124) ein Druckregler zur Regelung des Gasdruckes in Abhängigkeit vom Blutdruck ist.

7.  Blutoxygeniervorrichtung nach einem der vorhergehenden Ansprüche,
    **dadurch gekennzeichnet,**
    daß in dem Gehäuse (103) zusätzlich zu der Gasaustausch-Membran (16) auch ein Wärmetauscher (105) im Weg des Blutstromes (62) in Wärmetausch mit diesem Blutstrom angeordnet ist.

8.  Blutoxygeniervorrichtung nach einem der vorhergehenden Ansprüche,
    **dadurch gekennzeichnet,**
    daß im Blutweg nach der Gasaustausch-Membran (16) eine Strömungsdrossel (200) angeordnet ist, deren Strömungsquerschnitt in Abhängigkeit vom Blutdruck variabel ist, wobei der Strömungsquerschnitt mit abnehmendem Blutdruck kleiner wird bzw. mit zunehmendem Blutdruck größer.

FIG.1

EP 0 956 871 A2